# EUROPEAN PATENT APPLICATION

(11) **EP 3 015 046 A1**
(43) Date of publication of application: **04.05.2016**
(21) Application number: 14816934.5
(22) Date of filing: 10.06.2014
(51) Int. Cl.: A61B 1/00, G02B 23/24

(54) **SPIRAL CAP, CAP UNIT, SPIRAL UNIT AND GUIDE DEVICE**

(30) Priority: 26.06.2013 US 201361839510 P
(71) Applicant: OLYMPUS CORPORATION, Shibuya-ku Tokyo 151-0072 (JP)
(72) Inventor: AILINGER, Robert E., Massachusetts 01772-2104 (US); FRASSICA, James J., Massachusetts 01772-2104 (US); ANDREWS, Richard M., Massachusetts 01772-2104 (US); MIYOSHI, Hiroaki, Tokyo 151-0072 (JP)
(74) Representative: Schicker, Silvia
(86) International application number: PCT/JP2014/065378
(87) International publication number: WO 2014/208334

(57) **Abstract**

A spiral cap includes: a cap body; a coupling portion provided on the cap body and coupled to an end portion of a tube body which is rotatably attached to an insertion section of an introducing device; a fin connector to which an end portion of a main fin portion is connected, the main fin portion being provided on an outer surface of the tube body in a spiral shape about a longitudinal axis of the tube body; and an insertion assisting fin portion provided on an outer surface of the cap body and forming a fin in cooperation with the main fin portion connected to the fin connector.

## Description

### Technical Field

The present invention relates to a spiral cap and a cap unit for a spiral unit which is rotatably attached to an insertion section having a longitudinal axis, and relates to a spiral unit including the cap unit, and an introducing device including the spiral unit.

### Background Art

For example, US 2012/0029281 A1 discloses a spiral unit through which an insertion section is inserted. The spiral unit can be rotated in two directions (circumferential directions) about a longitudinal axis of the insertion section. Thus, by rotating the spiral unit in a proper direction relative to the insertion section, a distal end of the insertion section can be moved to the depth side or near side of a duct.

In an example of the work of manufacturing such a spiral unit, a fin is disposed and fixed by an adhesive to an outer surface of a tube body that serves as a base, such that the fin is formed spiral with an equal pitch in the longitudinal direction and the height direction of the fin is defined in a radially outward direction of the tube body.

In the work of manufacturing the spiral unit as disclosed, for example, in US 2012/0029281 A1, when an end portion of the spiral fin is fixed to the tube body, it is necessary to define the height of the fin such that the height of the fin gradually increases away from the end portion of the tube body. Thus, the end portion of the fin is cut in advance with high precision in a curved-surface shape corresponding to the curved outer surface of the tube body, and then the cut surface of the fin is manually fixed to the outer surface of the tube. At this time, time and high skill are needed in order to cut the fin in the curved-surface shape with high precision, and to precisely position, adhere and fix the cut surface to the outer surface of the tube. It is difficult, therefore, to mass-produce proper spiral units.

### Summary of Invention

The object of this invention is to provide a spiral cap and a cap unit for a spiral unit which can shorten a manufacturing time, is easy to manufacture without high skill, and is suited to mass-production, and to provide a spiral unit including the cap unit, and an introducing device including the spiral unit.

According to one aspect of the present invention, a spiral cap includes: a cap body; a coupling portion provided on the cap body and coupled to an end portion of a tube body which is rotatably attached to an insertion section of an introducing device; a fin connector to which an end portion of a main fin portion is connected, the main fin portion being provided on an outer surface of the tube body in a spiral shape about a longitudinal axis of the tube body; and an insertion assisting fin portion provided on an outer surface of the cap body and forming a fin in cooperation with the main fin portion connected to the fin connector.

### Brief Description of Drawings

FIG. 1 is a schematic view illustrating an endoscope (introducing device) on which a spiral unit according to first to third embodiments is mounted, and a peripheral unit thereof.
FIG. 2 is a longitudinal cross-sectional view which schematically illustrates the structure of a second relay connection section of an insertion section of the endoscope on which the spiral unit according to the first to third embodiments is mounted.
FIG. 3 is a schematic transverse cross-sectional view, taken along line III-III in FIG. 2, illustrating the second relay connection section of the insertion section of the endoscope on which the spiral unit according to the first to third embodiments is mounted.
FIG. 4 is a schematic transverse cross-sectional view, taken along line IV-IV in FIG. 2, illustrating the second relay connection section of the insertion section of the endoscope on which the spiral unit according to the first to third embodiments is mounted.
FIG. 5A is a schematic perspective view illustrating a state before fitting a tube body to a cap unit in a case of manufacturing the spiral unit according to the first embodiment.
FIG. 5B is a schematic side view illustrating a state before fitting the tube body to the cap unit in the case of manufacturing the spiral unit according to the first embodiment.
FIG. 5C is a schematic side view illustrating a state in which the tube body is fitted to the cap unit and a distal end of a main fin portion is positioned in the case of manufacturing the spiral unit according to the first embodiment, and in which a bottom surface (band portion) of the main fin portion is put in contact with an outer surface of the tube body, the height direction of the main fin portion is defined in a radially outward direction of the tube body, and the main fin portion is disposed with an equal pitch in the longitudinal direction of the tube body.
FIG. 5D is a schematic side view illustrating a state in which a spiral cap that is the same as the spiral cap provided at a distal end portion of the spiral unit according to the first embodiment is disposed at a proximal end portion of the spiral unit.
FIG. 6 is a schematic perspective view illustrating the main fin portion of the spiral unit according to the first embodiment.
FIG. 7 is a schematic longitudinal cross-sectional view illustrating a state in which the main fin portion is fixed to the outer surface of the tube body of the spiral unit according to the first embodiment.
FIG. 8A is a schematic view illustrating, from a front side of the spiral cap, a state in which the main fin portion of the spiral unit according to the first embodiment is fitted in a fin connector which is provided on an insertion assisting fin portion.
FIG. 8B is a schematic transverse cross-sectional view, taken along line 8B-8B in FIG. 5B and FIG. 8A, illustrating the insertion assisting fin portion of the spiral unit according to the first embodiment.
FIG. 9A is a schematic perspective view illustrating a state before fitting a tube body to a cap unit in a case of manufacturing the spiral unit according to the second embodiment.
FIG. 9B is a schematic transverse cross-sectional view illustrating a state in which an opening is formed in a side surface of the insertion assisting fin portion of the cap unit of the spiral unit according to the first embodiment.
FIG. 10 is a schematic perspective view illustrating the main fin portion of the spiral unit according to the second embodiment.
FIG. 11A is a schematic side view illustrating a state in which the tube body is fitted to the cap unit and a distal end of the main fin portion is positioned in the case of manufacturing the spiral unit according to the second embodiment, and in which a bottom surface (band portion) of the main fin portion is put in contact with an outer surface of the tube body, the height direction of the main fin portion is defined in a radially outward direction of the tube body, and the main fin portion is disposed with an equal pitch in the longitudinal direction of the tube body.
FIG. 11B is a schematic longitudinal cross-sectional view illustrating a state in which the main fin portion is fixed to the outer surface of the tube body of the spiral unit according to the second embodiment.
FIG. 12 is a schematic perspective view illustrating a spiral cap of a spiral unit according to the third embodiment.
FIG. 13 is a schematic longitudinal cross-sectional view illustrating a state in which a distal end of the main fin portion is fitted on a projection portion of a fin connector of the spiral unit according to the third embodiment.
FIG. 14 is a schematic transverse cross-sectional view illustrating a second relay connection section of an insertion section, FIG. 14 illustrating a modification of the insertion section of the endoscope on which the spiral unit according to the first to third embodiments is mounted.

### Description of Embodiments

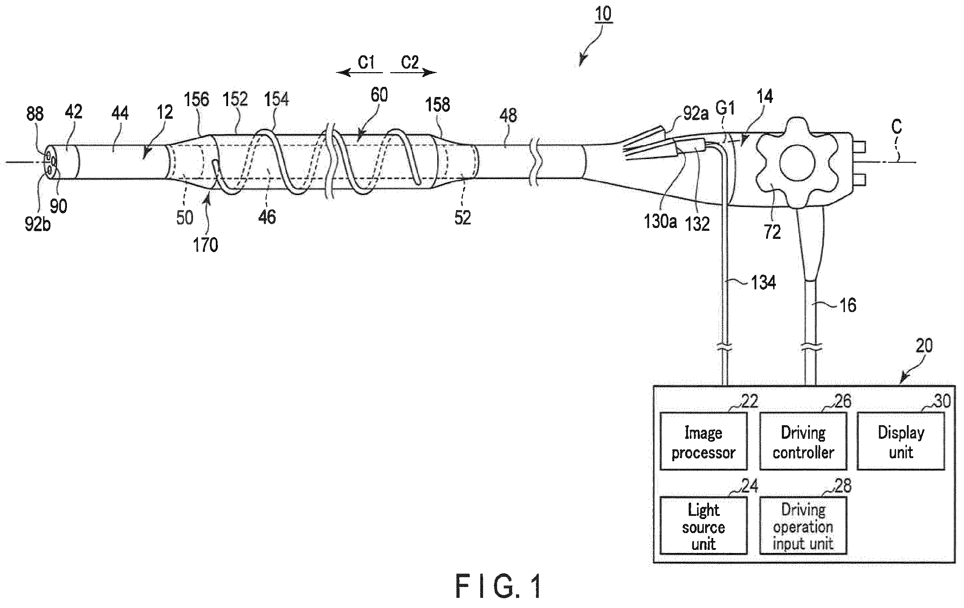

Embodiments of the invention will be described hereinafter with reference to the accompanying drawings, that is, FIG. 1 to FIG. 14.

A first embodiment is described with reference to FIG. 1 to FIG. 8B.

As illustrated in FIG. 1, an endoscope (an introducing device for various ducts) 10 has a longitudinal axis (center axis) C. One of directions parallel to the longitudinal axis C (a direction of arrow C1 in FIG. 1) is a distal-end direction, and an opposite direction to the distal-end direction C1 (a direction of arrow C2 in FIG. 1) is a proximal-end direction. The endoscope 10 includes an insertion section (endoscope insertion section) 12 which is provided to extend along the longitudinal axis C; an operation section (endoscope operation section) 14 which is provided on the proximal-end direction side of the insertion section 12; and a spiral unit 60 which is mounted on an outer periphery of the insertion section 12. The insertion section 12 is provided to extend along the longitudinal axis C, and is inserted into a duct from a distal end thereof at a time when the endoscope 10 is used.

A universal cable 16 extends from the operation section 14. Of the universal cable 16, a distal end thereof relative to the operation section 14 is connectable to a peripheral unit 20. The peripheral unit 20 includes, for example, an image processor 22, a light source unit 24, a driving controller 26, a driving operation input unit 28, and a display unit 30.

The insertion section 12 includes a distal rigid section 42 which is provided at a part located most on the distal-end direction side; a bending section 44 which is provided on the proximal-end direction side of the distal rigid section 42; a first flexible section 46 which is provided on the proximal-end direction side of the bending section 44; and a second flexible section 48 which is provided on the proximal-end direction side of the first flexible section 46. The bending section 44 and first flexible section 46 are connected by a first relay connection section 50. The first flexible section 46 and second flexible section 48 are connected by a second relay connection section 52.

The spiral unit 60 is provided to extend along the longitudinal axis C, for example, between the first relay connection section 50 and the second relay connection section 52. The spiral unit 60 is attached to the insertion section 12 in a state in which the insertion section 12 is inserted through the spiral unit 60. In the present embodiment, the spiral unit 60 is rotatable about the longitudinal axis C relative to the insertion section 12.

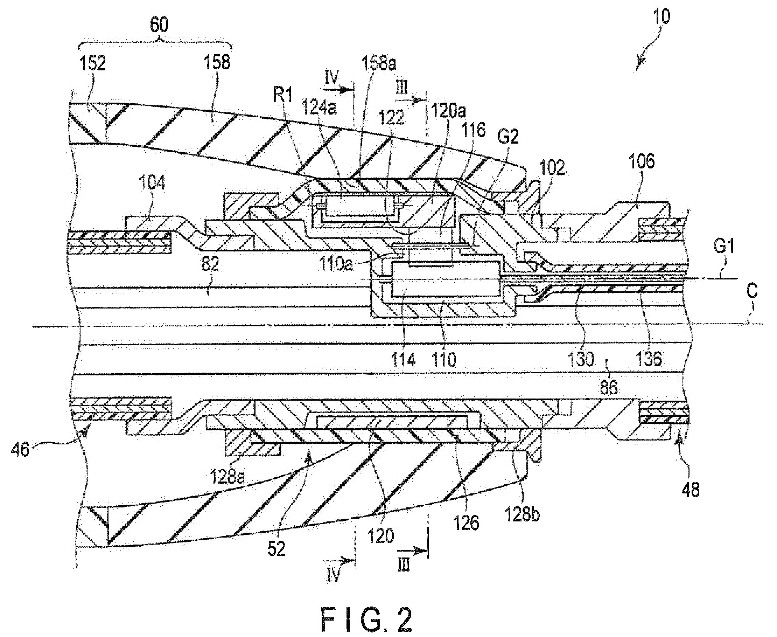

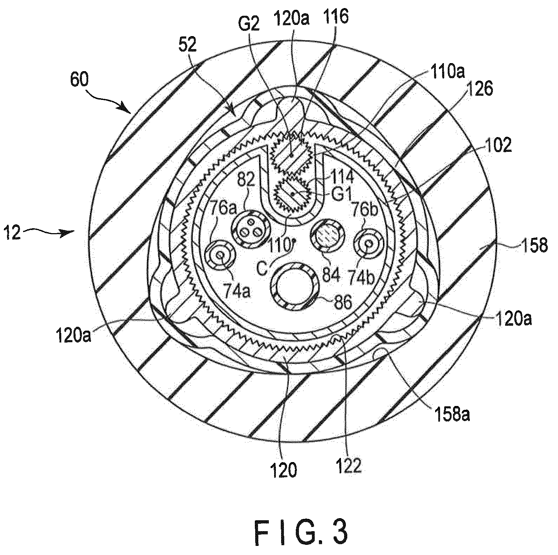

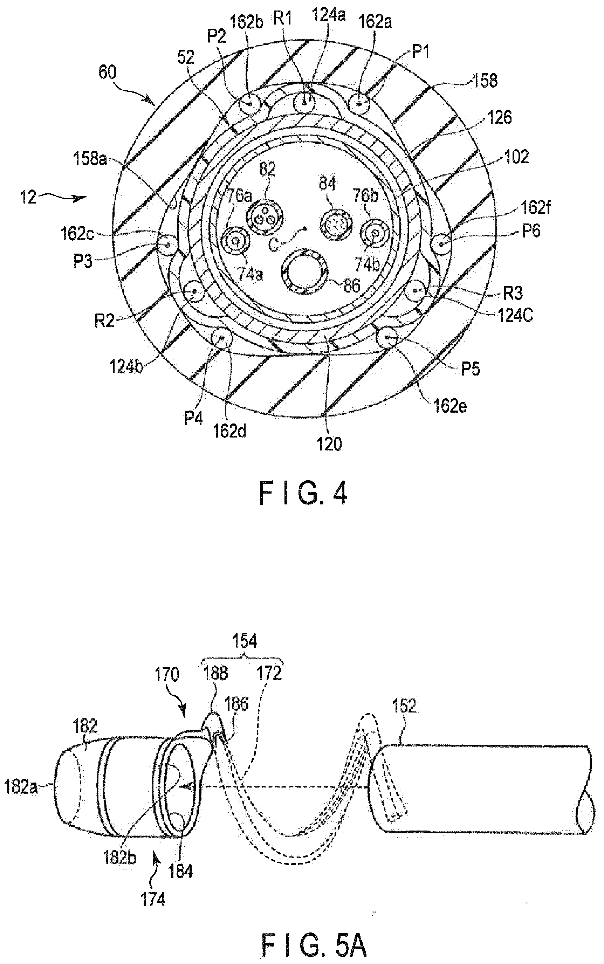

FIG. 2 illustrates the structure of the second relay connection section 52. FIG. 3 is a transverse cross-sectional view taken along line III-III in FIG. 2, and FIG. 4 is a transverse cross-sectional view taken along line IV-IV in FIG. 2.

As illustrated in FIG. 1, a bending operation knob 72, which is a bending operation input unit to which a bending operation of the bending section 44 is input, is provided on the outer surface of the operation section 14. As illustrated in FIG. 3 and FIG. 4, bending wires 74a and 74b and coils 76a and 76b, through which the bending wires 74a and 74b are passed, are provided to extend along the longitudinal axis C within the insertion section 12. Distal ends of the coils 76a and 76b are connected to, for example, an inner peripheral surface of the first relay connection section 50. In the inside of the operation section 14, proximal ends of the bending wires 74a and 74b are connected to a pulley (not shown) which is coupled to the bending operation knob 72. Distal ends of the bending wires 74a and 74b are connected to, for example, a distal end portion of the bending section 44. By the operation of the bending operation knob 72, the bending wire 74a or bending wire 74b is pulled, and the bending section 44 is bent in a desired direction.

In the meantime, in the present embodiment, the two bending wires 74a and 74b are provided and the bending section 44 can be bent in two directions. However, for example, four bending wires may be provided and the bending section 44 may be bent in four directions.

As illustrated in FIG. 2 to FIG. 4, an observation optical system, an illumination optical system and a channel are provided within the insertion section 12. To be more specific, within the insertion section 12, an imaging cable 82, a light guide 84 and a treatment instrument channel tube 86 are provided to extend along the longitudinal axis C. An imaging element (not shown) for imaging a subject is provided within the distal rigid section 42 (distal end portion of insertion section 12). The imaging element images a subject through an observation window 88. A distal end of the imaging cable 82 is connected to the imaging element. The imaging cable 82 is provided to extend through the insertion section 12, operation section 14 and universal cable 16, and a proximal end of the imaging cable 82 is connected to the image processor 22 of the peripheral unit 20. The image processor 22 generates an image of the subject. The generated image of the subject is displayed on the display unit 30.

The light guide 84 is provided to extend through the insertion section 12, operation section 14 and universal cable 16, and a proximal end of the light guide 84 is connected to the light source unit 24 of the peripheral unit 20. Light, which is emitted from the light source unit 24, is guided by the light guide 84 and radiated on the subject from an illumination window 90 at the distal end portion (distal rigid section 42) of the insertion section 12.

As illustrated in FIG. 1, a treatment instrument insertion portion 92a, through which a treatment instrument, such as a forceps, is inserted, is provided on the outer surface of the operation section 14. The treatment instrument channel tube 86 is passed through the insertion section 12 and operation section 14, and a proximal end thereof is connected to the treatment instrument insertion portion 92a. The treatment instrument, which is inserted from the treatment instrument insertion portion 92a, is passed through the treatment instrument channel tube 86, and projects toward the distal-end direction from an opening portion 92b of the distal rigid section 42. In addition, in the state in which the treatment instrument projects from the opening portion 92b of the distal rigid section 42, treatment by the treatment instrument is performed.

As illustrated in FIG. 2, the second relay connection section 52 includes a base member 102. A proximal end portion of the first flexible section 46 is coupled to a distal end portion of the base member 102 via a relay member 104. Thus, the first flexible section 46 and second relay connection section 52 are coupled. A distal end portion of the second flexible section 48 is coupled to a proximal end portion of the base member 102 via a relay member 106. Thus, the second flexible section 48 and second relay connection section 52 are coupled.

As illustrated in FIG. 2 to FIG. 4, the second relay connection section 52 has a hollow portion 110 in the base member 102. The hollow portion 110 is open to the outside at an opening portion 110a. In addition, a driving gear 114 and a relay gear 116 are attached to the base member 102. The driving gear 114 is disposed in the hollow portion 110, and the relay gear 116 is disposed near the opening portion 110a of the hollow portion 110. The driving gear 114 is meshed with the relay gear 116. The driving gear 114 is rotatable about a driving axis G1. The relay gear 116 is rotatable about a gear axis G2.

A rotary cylindrical member 120 having a cylindrical shape is attached to the base member 102 of the second relay connection section 52. The rotary cylindrical member 120 is rotatable about the longitudinal axis C relative to the insertion section 12 (base member 102). An inner peripheral gear portion 122 is disposed over the entire periphery in a direction about the longitudinal axis C. The inner peripheral gear portion 122 of the rotary cylindrical member 120 is meshed with the relay gear 116.

The rotary cylindrical member 120 includes a roller support portion 120a by which, for example, three inside rollers 124a, 124b and 124c are supported. The inside rollers 124a, 124b and 124c are disposed substantially at regular intervals in a direction (circumferential direction) about the longitudinal axis C. The inside rollers 124a, 124b and 124c have corresponding roller axes R1, R2 and R3, respectively. The inside rollers 124a, 124b and 124c are rotatable relative to the rotary cylindrical member 120, respectively, about the corresponding roller axes R1, R2 and R3. In addition, the inside rollers 124a, 124b and 124c are rotatable, as one piece with the rotary cylindrical member 120, relative to the insertion section 12 (base member 102) about the longitudinal axis C.

A cylindrical cover member 126 covers the outside of the rotary cylindrical member 120 and inside rollers 124a, 124b and 124c. A distal end of the cover member 126 is fixed to the base member 102 by an annular engaging member 128a. Liquid-tightness is kept between the base member 102 and cover member 126 by the engaging member 128a at the distal end of the cover member 126. A proximal end of the cover member 126 is fixed to the base member 102 by an annular engaging member 128b. Liquid-tightness is kept between the base member 102 and cover member 126 by the engaging member 128b at the proximal end of the cover member 126. Thus, a liquid is prevented from entering the hollow portion 110, rotary cylindrical member 120 and inside rollers 124a, 124b and 124c, which are located inside the cover member 126.

As illustrated in FIG. 3 and FIG. 4, the cover member 126 projects outward at locations where the inside rollers 124a, 124b and 124c are positioned in the direction about the longitudinal axis C. In the meantime, while the cover member 126 is fixed to the outside of the base member 102, that is, while the cover member 126 is fixed to the outer periphery of the insertion section 12, the rotary cylindrical member 120 is rotatable about the longitudinal axis C relative to the cover member 126.

As illustrated in FIG. 1, the operation section 14 includes, on the outer surface thereof, a proximal-end opening 130a of a channel 130 through which a driving shaft 136 (to be described later) is passed. A motor 132, which is a driving member, is attached to the proximal-end opening 130a of the channel 130. One end of a motor cable 134 is connected to the motor 132. The other end of the motor cable 134 is connected to the driving controller 26 of the peripheral unit 20.

As illustrated in FIG. 2, a driving shaft 136, which is a line-shaped member, is provided to extend along a driving axis G1 within the second flexible section 48 of the insertion section 12. A distal end of the driving shaft 136 is connected to the driving gear 114. A proximal end of the driving shaft 136 is connected to the motor 132 which is attached to the proximal-end opening 130a of the channel 130. In addition, a distal end of the channel 130 is connected to the base member 102 so as to communicate with the hollow portion 110. A proximal end of the channel 130 is connected to the proximal-end opening 130a. The driving shaft 136 is provided to extend through the channel tube 130.

By an operational input in the driving operation input unit 28, the driving controller 26 supplies power to the motor 132 via the motor cable 134, and controls the driving of the motor 132. By driving the motor 132, the driving controller 26 causes the driving shaft 136 to generate a rotational driving force which rotates the driving shaft 136. Thus, the driving shaft 136 and driving gear 114 rotate about the driving axis G1. Here, the driving axis G1 passes through the center of the driving gear 114 and driving shaft 136, and is substantially parallel to the longitudinal axis C within the second flexible section 48. In addition, the driving axis G1 is bent toward the proximal-end opening 130a of the channel tube 130 within the operation section 14.

The driving gear 114 rotates about the driving axis G1, and thereby the relay gear 116, which is meshed with the driving gear 114, rotates about the gear axis G2. The rotary cylindrical member 120 rotates about the longitudinal axis C by the inner peripheral gear portion 122 which is meshed with the relay gear 116. Specifically, the rotational driving force of the motor 132 is transmitted to the driving shaft 136, driving gear 114, relay gear 116 and rotary cylindrical member 120. Thus, if the rotary cylindrical member 120 rotates about the longitudinal axis C, the inside rollers 124a, 124b and 124c, which are supported by the rotary cylindrical member 120, move in a direction about the longitudinal axis C relative to the insertion section 12 and cover member 126.

As illustrated in FIG. 1, the spiral unit 60 includes a tube body 152 which serves as a base, a spiral fin 154 which is attached to an outer surface of the tube body 152, a distal-side taper portion 156 having a tubular shape which is provided on the distal end side of the tube body 152, and a cylindrical proximal-side taper portion 158 which is provided on the proximal end side of the tube body 152.

The spiral unit 60 according to this embodiment is, when used, mounted on the outer periphery of the first flexible section 46 of the insertion section 12, each time the endoscope 10 is used.

The tube body 152, on which the spiral fin 154 is attached, is formed of, for example, a thermoplastic resin. The spiral fin 154 is provided along a fin axis F which is provided to extend spirally about the longitudinal axis C. The inner periphery of the tube body 152 is so formed as to be able to pass the distal rigid section 42, bending section 44 and first flexible section 46 of the insertion section 12.

The distal-side taper portion 156 is formed in a taper shape with an outside diameter decreasing toward the distal-end direction side. The proximal-side taper portion 158 is formed in a taper shape with an outside diameter decreasing toward the proximal-end direction side. Thus, when the insertion section 12 is inserted or drawn out in/from a duct in the state in which the spiral unit 60 is attached to the outer surface of the insertion section 12, the distal end and proximal end of the spiral unit 60 are prevented as much as possible from being caught on the inner peripheral surface of the duct.

As illustrated in FIG. 4, six outside rollers 162a, 162b,..., 162f are attached to an inner peripheral surface 158a of the proximal-side taper portion 158. The outside rollers 162a, 162b,..., 162f are disposed on the outside of the cover member 126. In the direction (circumferential direction) about the longitudinal axis C, the inside roller 124a is disposed between the two outside rollers 162a and 162b, and the inside roller 124b is disposed between the outside rollers 162c and 162d. In addition, in the direction (circumferential direction) about the longitudinal axis C, the inside roller 124c is disposed between the outside rollers 162e and 162f. The respective outside rollers 162a, 162b,..., 162f have corresponding roller axes P1, P2,..., P6. The respective outside rollers 162a, 162b,..., 162f are rotatable about the corresponding roller axes P1, P2,..., P6, relative to the cover member 126 and proximal-side taper portion 158.

In this embodiment, the inner peripheral surface of the proximal-side taper portion 158 has a shape other than a circular shape. In addition, the rotary cylindrical member 120, on the outside of which the inside rollers 124a, 124b and 124c are supported and on the outside of which the cover member 126 is fixed, is fitted, in the direction (circumferential direction) about the longitudinal axis C, to the inner peripheral surface 158a of the proximal-side taper portion 158, on the inner peripheral surface of which the outside rollers 162a, 162b,..., 162f are attached.

In the meantime, the positional relationship between the respective rollers 124a to 124c and 162a to 162f is not limited to the above-described state. For example, it is preferable that the inside roller 124b or inside roller 124c is disposed between the outside roller 162a and 162b, it is preferable that the inside roller 124c or inside roller 124a is disposed between the outside roller 162c and 162d, and it is preferable that the inside roller 124a or inside roller 124b is disposed between the outside roller 162e and 162f.

Thus, if the rotary cylindrical member 120 rotates, as described above, by the driving of the motor 132, the inside roller 124a pushes, based on the rotational direction, the outside roller 162a or outside roller 162b. Similarly, the inside roller 124b pushes the outside roller 162c or outside roller 162d, and the inside roller 124c pushes the outside roller 162e or outside roller 162f. Thus, the rotational driving force of the motor 132 is transmitted from the inside rollers 124a, 124b and 124c to the outside rollers 162a to 162f, that is, to the spiral unit 60. Accordingly, the spiral unit 60 including the tube body 152, to which the fin 154 is attached, rotates about the longitudinal axis C relative to the insertion section 12 and cover member 126.

Thus, the outside rollers 162a, 162b,..., 162f can rotate, together with the spiral unit 60, about the longitudinal axis C relative to the insertion section 12 (base member 102).

In the meantime, the respective inside rollers 124a, 124b and 124c rotate about the corresponding roller axes R1, R2 and R3. Thus, the friction between the inside rollers 124a, 124b and 124c and the cover member 126 decreases. Similarly, since the respective outside rollers 162a, 162b,..., 162f rotate about the corresponding roller axes P1, P2,..., P6, the friction between the outside rollers 162a, 162b,..., 162f and the outer surface of the cover member 126 decreases. Hence, the rotational driving force is properly transmitted from the inside rollers 124a, 124b and 124c, which are supported on the rotary cylindrical member 120, to the spiral unit 60, and the spiral unit 60 properly rotates relative to the base member 102 which the second relay connection 52 of the insertion section 12 includes. Since the spiral unit 60 (tube body 152 and fin 154) rotates relative to the insertion section 12 in the state in which the spiral fin 154 is in contact with a wall part such as an inner wall of a duct, an impelling force toward the distal-end direction C1 or proximal-end direction C2 along the longitudinal axis C acts on the insertion section 12 to which the spiral unit 60 is attached.

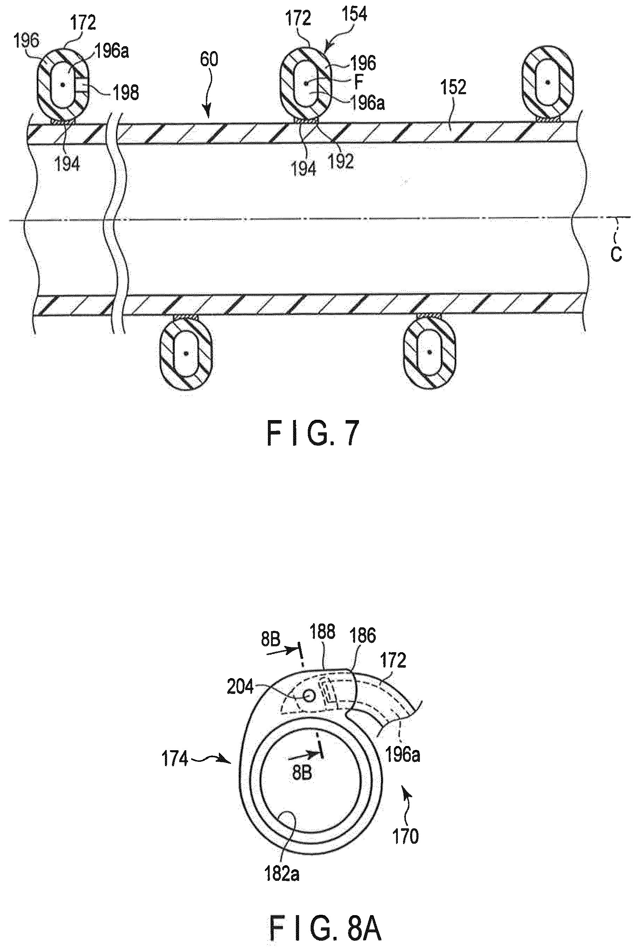

Here, referring to FIG. 5A to FIG. 8B, a description is given of the structure of the distal-side taper portion 156 of the spiral unit 60.

In this embodiment, the spiral unit 60 includes a cap unit 170 including the spiral fin 154. The cap unit 170 according to this embodiment is disposed at the distal end of the tube body 152.

The cap unit 170 includes a main fin portion (first fin portion) 172 and a spiral cap 174. The spiral cap 174 includes a cap body 182, a coupling portion 184 to which the distal end of the tube body 152 is coupled, a fin connector 186 to which a distal end of the main fin portion 172 is connected, and an insertion assisting fin portion (second fin portion) 188. In addition, an end portion of the main fin portion 172 is fitted and connected to the fin connector 186, thus forming the spiral fin 154 together with the insertion assisting fin portion 188.

The cap body 182 of the spiral cap 174 shown in FIG. 5A to FIG. 5C is formed in a cylindrical shape having openings 182a and 182b which are formed along the longitudinal axis C at a distal end and a proximal end thereof. Specifically, the cap body 182 has a through-hole which is defined by the openings 182a and 182b and an inner peripheral surface thereof. The inner peripheral surface and outer surface (outer peripheral surface) of the cap body 182 are formed in a taper shape which is thinner at the distal end than at the proximal end. The coupling portion 184, to which the distal end of the tube body 152 is coupled, is disposed at the proximal end portion of the cap body 182, and is formed such that the distal end portion of the tube body 152 is, for example, fitted to, and fixed by adhesion or welding to, the coupling portion 184. As an adhesive that is used for adhesion between the coupling portion 184 of the spiral cap 174 and the tube body 152, use is made of, for instance, an ultraviolet-curing adhesive that is cured by irradiation of ultraviolet.

It is preferable that the insertion assisting fin portion 188 is formed integral with the cap body 182. The insertion assisting fin portion 188 is provided on an outer surface of the cap body 182 of the spiral cap 174, and is formed in a state in which the insertion assisting fin portion 188 projects in a radially outward direction of the longitudinal axis C relative to the outer surface of the cap body 182. The insertion assisting fin portion 188 is formed in a spiral shape so as to constitute a distal end of the spiral fin 154, that is, a part of the spiral fin 154. A rear-side end portion of the insertion assisting fin portion 188 may be located on the distal end side of the rear end of the cap body 182, or may be located on the rear side of the rear end of the cap body 182. In this embodiment, a description is given on the assumption that the rear-side end portion of the insertion assisting fin portion 188 is located on the rear side of the rear end of the cap body 182.

In the meantime, it is preferable that a spiral-shaped insertion assisting fin portion 188, which is provided with a fin connector 186 and to which an end portion (proximal end) of the main fin portion 172 is fitted, is also formed on the outer surface of the proximal-side taper portion 158 of the spiral unit 60, like the outer surface of the distal-side taper portion 156 of the spiral unit 60.

The fin connector 186 is used for connecting the end portion (distal end) of the main fin portion 172 to the rear-side end portion of the insertion assisting fin portion 188. The fin connector 186 is provided at the rear-side end portion of the insertion assisting fin portion 188. It is preferable that the insertion assisting fin portion 188 is formed integral with the fin connector 186. The fin connector 186 is formed between the outer surface of the spiral cap 174 and the outer surface of the insertion assisting fin portion 188, depending on the length of the insertion assisting fin portion 188. It is preferable that the transverse cross section of the fin connector 186 according to this embodiment is formed in a circumferentially discontinuous shape, such as a substantially U shape or a substantially C shape, so that the distal end of the main fin portion 172 may easily be fitted on the outside of the tube body 152.

In addition, the distal end of the main fin portion 172 is fitted to the fin connector 186. Specifically, the distal end of the main fin portion 172 is fitted and coupled to the fin connector 186. Thus, the distal end of the main fin portion 172 is continuous with the insertion assisting fin portion 188. When the distal end of the main fin portion 172 has been fitted and coupled to the fin connector 186, the main fin portion 172 and insertion assisting fin portion 188 cooperate to form the spiral fin 154.

In the meantime, it is preferable that the distal end of the main fin portion 172 is fixed to the fin connector 186 by an adhesive or the like.

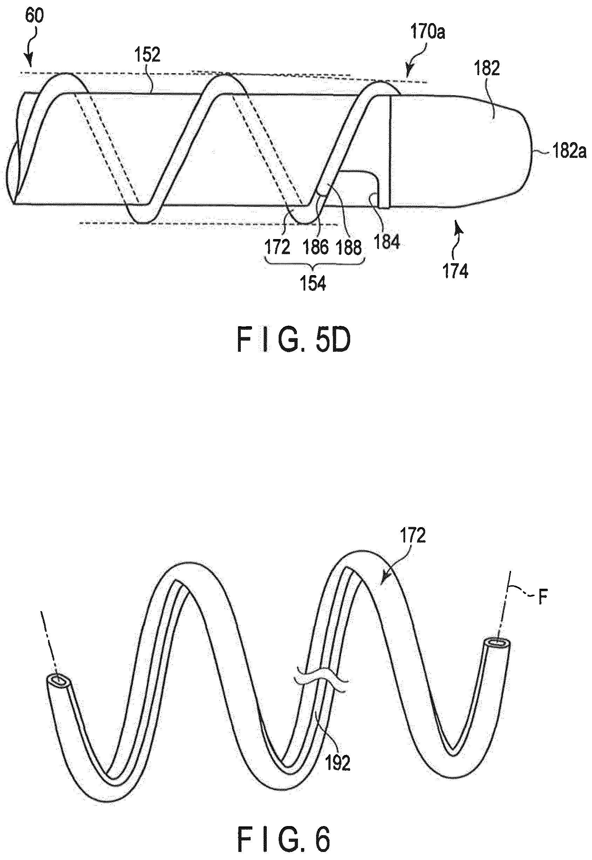

Here, the main fin portion 172 illustrated in FIG. 6 is formed by flattening, from one end to the other end, a tube formed of a band-shaped, soft, elastic resin material such as silicone, and bending the tube in a spiral shape. Specifically, the main fin portion 172 is held in a spirally reformed state.

As illustrated in FIG. 7, in a state in which the main fin portion 172 is fixed to the outer surface of the tube body 152, the main fin portion 172 includes an annular wall 196 which forms a cavity section 196a. In addition, the main fin portion 172 is formed in a tube shape extending along the fin axis F. Accordingly, the annular wall 196 and cavity section 196a extend along the fin axis F. Here, since the fin axis F is provided to extend spirally about the longitudinal axis C, the hollow portion 196a is also provided to extend spirally about the longitudinal axis C. Incidentally, in the present embodiment, the fin axis F agrees with the center axis of the cavity section 196a.

The length of the main fin portion 172 along the fin axis F is greater than the entire length of the tube body 152 along the longitudinal axis C, since the main fin portion 172 is spirally fixed to the outer surface of the tube body 152 from the distal end to proximal end of the main fin portion 172. If the proximal end of the main fin portion 172 is moved along the longitudinal axis C in the state in which the distal end of the main fin portion 172 is coupled to the fin connector 186, the pitch of the main fin portion 172 relative to the outer surface of the tube body 152 can easily be set at a proper pitch by the elastic function of the main fin portion 172. A strip portion 192 (see FIG. 6) of the main fin portion 172, which forms the bottom surface thereof, is fixed by an adhesive or the like indicated by reference numeral 194, as illustrated in FIG. 7. At this time, since the pitch of the main fin portion 172 can easily be set by the elastic function of the main fin portion 172, the height direction of the main fin portion 172, that is, the spiral radius, can easily be set in a direction perpendicular to the longitudinal axis C.

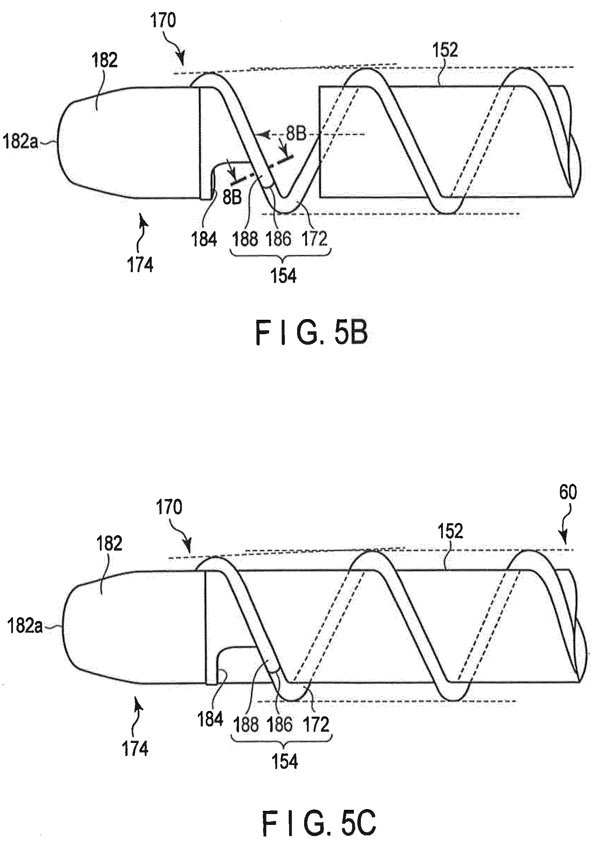

As illustrated in FIG. 5B and FIG. 5C, the insertion assisting fin portion 188 is formed such that the fin height relative the outer surface of the spiral cap 174 becomes smaller toward the distal end side of the insertion assisting fin portion 188. In other words, the insertion assisting fin portion 188 is formed such that the fin height of the insertion assisting fin portion 188 increases from the outer surface of the spiral cap 174 toward the proximal end of the spiral cap 174. Specifically, the insertion assisting fin portion 188 is formed such that the fin height of the insertion assisting fin portion 188 increases from the outer surface of the cap body 182 toward the fin connector 186. Thereby, the fin height of the insertion assisting fin portion 188 gradually increases from the outer surface of the spiral cap 174 toward the distal end portion of the main fin portion 172 which is coupled to the fin connector 186, and substantially reaches the height of the main fin portion 172 at the position of the fin connector 186. Thus, when the insertion section 12 is inserted into that part of a duct, which steeply decreases in diameter (e.g. pylorus), it is possible to abut the distal end of the distal end of the spiral fin 154 upon the inner wall of the part with the decreased diameter, and to easily insert the spiral unit 60 into the duct.

In the meantime, as illustrated in FIG. 5D, a cap unit 170a, which has the same structure as the cap unit 170, may be used for the distal-side taper portion 158 that is disposed at the proximal end of the tube body 152. Specifically, the proximal end portion of the insertion assisting fin portion 188 may adopt the same structure as the distal end portion of the insertion assisting fin portion 188 of the distal-side taper portion 156. In this case, the insertion assisting fin portion 188 of the cap unit 170a of the proximal-side taper portion 158 is formed such that the fin height relative the outer surface of the spiral cap 174 becomes smaller toward the proximal end side of the insertion assisting fin portion 188. In other words, the insertion assisting fin portion 188 is formed such that the fin height of the insertion assisting fin portion 188 gradually increases from the outer surface of the spiral cap 174 toward the distal end of the spiral cap 174. The spiral radius of the spiral fin 154 is gradually increased from the proximal end to distal end thereof. Thus, when the insertion section 12 is drawn out from that part of a duct, which steeply decreases in diameter (e.g. cardia), it is possible to abut the proximal end of the spiral fin 154 upon the inner wall of the part with the decreased diameter, and to easily draw out the spiral unit 60 from the duct. Therefore, the insertion assisting fin portion 188 can function also as a draw-out assisting fin portion.

In addition, the distal end of the main fin portion 172 is fitted to the fin connector 186. Thereby, as regards a radially distal edge portion of the main fin portion 172 relative to the longitudinal axis C and a radially distal edge portion of the insertion assisting fin portion 188 relative to the longitudinal axis C, the radially distal edge portion of the insertion assisting fin portion 188 is located outside of the radially distal edge portion of the main fin portion 172. The spiral radius of the spiral fin 154 is gradually increased from the distal end toward proximal end side thereof by the insertion assisting fin portion 188. Thus, when the insertion section 12 is inserted into the duct, a stepped part between the insertion assisting fin portion 188 and main fin portion 172 can be ignored, and the outer surface of the spiral fin 154 can easily be abutted on the inner wall of the duct from the distal end toward proximal end of the spiral fin 154.

Accordingly, for example, when the insertion section 12 is inserted in the distal end direction C1 of the duct, it is possible to prevent as much as possible the distal end portion of the spiral fin 154 (in particular, the coupling portion between the distal end of the main fin portion 172 and the proximal end of the insertion assisting fin portion 188) from being caught on the inner wall of the duct.

In the meantime, it is preferable that the spiral radius is constant at a part of the outer surface of the central portion of the main fin portion 172 between the distal end and proximal end of the tube body 152.

As illustrated in FIG. 7, an opening 198 is formed in a distal end portion of the main fin portion 172, for example, in a side surface of the distal end portion of the main fin portion 172. The opening 198 communicates with the cavity section 196a through the annular wall 196. It is preferable that the opening 198 is formed not at a radially farthest position relative to the longitudinal axis C of the tube body 152, that is, not at a top portion of the main fin portion 172, but at a position displaced from this position, that is, at a side surface of the main fin portion 172. When the opening 198 is formed at the top portion of the main fin portion 172, the inner wall of the duct is easily caught on an edge portion of the opening 198. By contrast, when the opening 198 is formed at the side surface of the main fin portion 172, it is possible to decrease the frequency of the edge portion of the opening 198 being caught on the inner wall of the duct.

The opening 198 is used in order to cause air to escape from inside the spiral fin 154 when the spiral unit 60 is sterilized by EOG (ethylene oxide gas) in a vacuum atmosphere. Thus, even in a low-pressure atmosphere, the spiral unit 60 of this embodiment can prevent a rupture of the spiral fin 154.

For example, a position, with which a duct such as a digestive tract comes in positive contact, is a position in a range from a central portion to a distal end portion between the distal end and proximal end of the main fin portion 172. Thus, by forming the opening 198 at the distal end portion of the main fin portion 172, it is possible to prevent as much as possible the opening 198 from being caught on the inner wall of the duct.

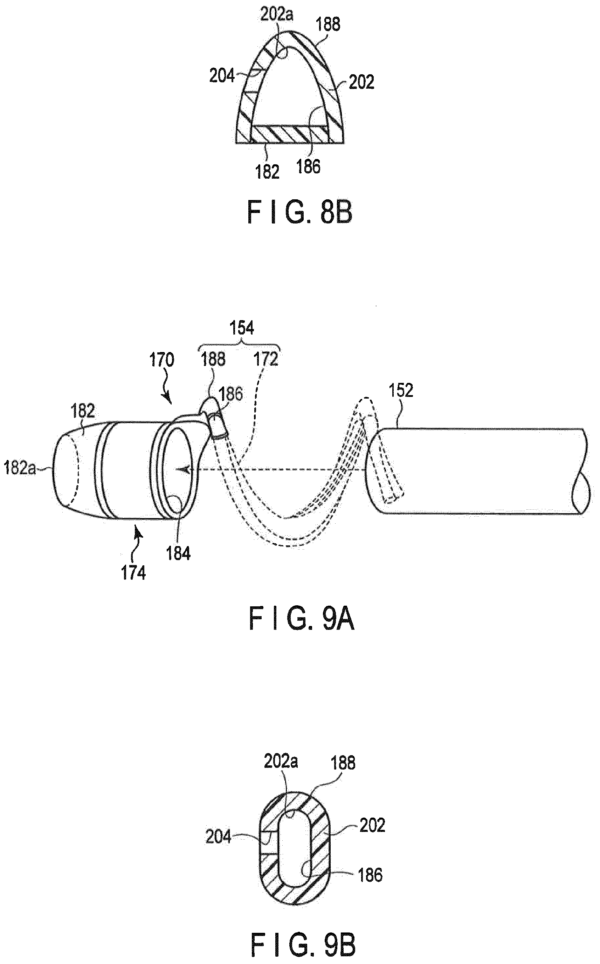

In the meantime, as illustrated in FIG. 8B, the insertion assisting fin portion 188 is formed to have a circumferentially discontinuous cross section such as a substantially U-shaped cross section or a substantially C-shaped cross section. The insertion assisting fin portion 188 includes a wall 202 which forms a hollow section 202a which can be engaged with an end portion of the main fin portion 172. Further, the inner wall of the wall 202 is formed to have such a curved-surface shape as to correspond to the outer surface of the tubular main fin portion 172. In addition, the insertion assisting fin portion 188 is formed to extend along the fin axis F. Accordingly, in the wall 202, the hollow portion 202a is provided to extend along the fin axis F. Here, since the fin axis F extends spirally about the longitudinal axis C, the hollow portion 202a also extends spirally about the longitudinal axis C. Incidentally, in the present embodiment, the fin axis F agrees with the center axis of the hollow portion 202a.

An opening 204 is formed in a side surface of the insertion assisting fin portion 188. The opening 204 communicates with the hollow portion 202a through the wall 202. It is preferable that the opening 204 is formed not at a farthest position, that is, a top portion, in the radial direction of the longitudinal axis C of the tube body 152 (the longitudinal axis extending from the distal end to proximal end of the spiral cap 174), but at a position displaced from this position, that is, at the side surface. When the opening 204 is formed at the top portion of the insertion assisting fin portion 188, the inner wall of the duct is easily caught on an edge portion of the opening 204. By contrast, when the opening 204 is formed at the side surface of the insertion assisting fin portion 188, it is possible to decrease the frequency of the edge portion of the opening 204 being caught on the inner wall of the duct.

The opening 204 communicates with the cavity section 196a of the main fin portion 172 that is coupled to the fin connector 186. The opening 204 is used in order to cause air to escape from inside the spiral fin 154 when the spiral unit 60 is sterilized by EOG (ethylene oxide gas) in a vacuum atmosphere. Thus, even in a low-pressure atmosphere, the spiral unit 60 of this embodiment can prevent a rupture of the spiral fin 154.

As described above, in the spiral unit 60, a position, with which a duct such as a digestive tract comes in positive contact, is a position in a range from the central portion to distal end portion of the main fin portion 172. Thus, by forming the opening 204 in the insertion assisting fin portion 188, that is, in the distal end portion of the spiral fin 154, it is possible to prevent as much as possible the opening 204 from being caught on the inner wall of the duct.

In addition, in this embodiment, the description has been given of the example in which the opening 198 is formed in the main fin portion 172, and the opening 204 is formed in the insertion assisting fin portion 188. However, it is preferable to form the opening in either the main fin portion 172 or the insertion assisting fin portion 188, if the cavity section 196a of the hollow section 196 of the main fin portion 172 communicates with the hollow portion 202a of the hollow portion 202 of the insertion assisting fin portion 188.

Besides, it is also preferable to form the opening 198, 204 in either the distal-end direction or proximal-end direction of the side surface of the spiral fin 154.

Next, the manufacturing process of the spiral unit 60 according to this embodiment is described.

When the spiral unit 60 is manufactured, as illustrated in FIG. 8A, the distal end of the tubular main fin portion 172, which is formed in a flat shape from the distal end to proximal end thereof and is formed in a spiral shape, is fitted to the fin connector 186 disposed in the insertion assisting fin portion 188 provided in the spiral cap 174, and thus the cap 170 is fabricated. At this time, it is preferable that the distal end of the main fin portion 172 and the fin connector 186 are fixed by an adhesive or the like.

The distal end of the tube body 152 is fitted to, and, where necessary, fixed by an adhesive to, the coupling portion 184 of the spiral cap 174 of the cap unit 170, and the band portion 192, which forms the bottom surface of the main fin portion 172 of the cap unit 170, is provisionally placed on the outer surface of the tube body 152.

When the insertion assisting fin portion 188 that is provided with the fin connector 186 is formed at the proximal-side taper portion 158, like the insertion assisting fin portion 188 of the distal-side taper portion 156, that is, when the proximal end of the tube body 152 is fitted to and, where necessary, fixed by an adhesive to, the coupling portion 184 of the spiral cap 174 of the cap unit 170a shown in FIG. 5D, the proximal end of the main fin portion 172 is fitted and fixed to the fin connector 186 of the proximal-side taper portion 158. Thus, the positions of the distal end and proximal end of the main fin portion 172 are determined.

Then, the disposition of the main fin portion 172 is adjusted relative to the outer surface of the tube body 152. In this state, the band portion 192 of the main fin portion 172 is fixed to the outer surface of the tube body 152 by using, for example, an ultraviolet-curing adhesive or the like. In particular, since the main fin portion 172 is spirally reformed in advance, the pitch of the main fin portion 172 in the direction along the longitudinal axis C can easily be adjusted relative to the outer surface of the tube body 152. Thus, a manufacturing worker of the spiral unit 60 can concentrate on making the height direction of the main fin portion 172 agree with the direction perpendicular to the longitudinal axis C.

In this manner, the position of the distal end of the main fin portion 172 is determined by the fin connector 186 which is provided on the insertion assisting fin portion 188 disposed on the spiral cap 174. Thus, the spiral fin 154 can easily be formed by simply fitting and adhering the end portion of the main fin portion 172, which is so cut as to fit to the fin connector 186, to the fin connector 186, and the manufacture of the spiral unit 60 can easily be proceeded with. Specifically, according to this manufacturing process, when the end portion of the spiral fin is fixed to the tube body, in order to define the height direction such that the height of the fin varies in a manner to gradually increase away from the end portion of the tube body, there is no need to perform such a time-consuming and skill-requiring procedure as precisely cutting an end portion of the spiral fin in advance in a curved-surface shape corresponding to the curved outer surface of the tube body, and precisely positioning, adhering and fixing the cut surface of the spiral fin to the outer surface of the tube body by a manual work.

Next, a description is given of an operation in a case in which the spiral unit 60 of the present embodiment is mounted on the endoscope 10 illustrated in FIG. 2 to FIG. 4.

The distal rigid section 42, bending section 44 and first flexible section 46 of the endoscope 10 are inserted into the proximal-side taper portion 158 of the thus formed spiral unit 60.

Then, the inner peripheral surface 158a of the proximal-side taper portion 158 of the spiral unit 60, which is formed between the outside rollers 162a and 162b, is fitted on the outer surface of the cover member 126 of the insertion section 12, which is raised by the inside roller 124a.

In this manner, the insertion section 12, on which the spiral unit 60 is mounted, is inserted into a duct. In the state in which the spiral fin 154 is abutted on the inner wall of the duct, the motor (driving member) 132 is driven, and the spiral unit 60 is rotated about the longitudinal axis C of the insertion section 12, as described above.

Specifically, in the state in which the spiral fin 154, which is provided to extend spirally about the longitudinal axis C, receives a pushing force from the inner wall of the duct along the longitudinal axis (center axis) C, the spiral unit 60 is rotated in one of the directions about the longitudinal axis C. By the spiral unit 60 rotating in one of the directions about the longitudinal axis C, an impelling force toward the distal-end direction C1 acts on the distal end of the insertion section 12.

In addition, in the state in which the spiral fin 154 receives a pushing force from the inner wall of the duct along the longitudinal axis (center axis) C, the spiral unit 60 (tube body 152 and spiral fin 154) is rotated in the other of the directions about the longitudinal axis C. By the spiral unit 60 rotating in the other of the directions about the longitudinal axis C, an impelling force toward the proximal-end direction C2 acts on the distal end of the insertion section 12.

In this manner, the efficiency in insertion of the insertion section 12 into the duct is improved by the impelling force toward the distal-end direction C1, and the efficiency in draw-out of the insertion section 12 from the duct is improved by the impelling force toward the proximal-end direction C2.

Since the spiral fin 154 is formed in a tubular shape including the hollow section 196, the spiral fin 154 has a proper elasticity. Since the spiral fin 154 has the proper elasticity, the occurrence of torsion and twist in the spiral fin 154 can effectively be prevented in the state in which the pushing force acts on the spiral fin 154 in the inner peripheral direction from the inner wall of the duct. Since no torsion or twist occurs in the spiral fin 154, the impelling force toward the distal-end direction C1 or proximal-end direction C2 properly acts on the insertion section 12 by rotating the spiral unit 60. Therefore, by rotating the spiral unit 60, the insertion and draw-out of the insertion section 12 in/from the duct is properly performed.

Since the spiral fin 154 is formed in the tubular shape including the hollow section 196, the spiral fin 154 has a proper flexibility. Thus, even in the state in which the spiral unit 60 is not rotated, the spiral fin 154 is easily bent by the pushing force from the inner wall of the duct by moving the insertion section 12 in the duct in the proximal-end direction. Therefore, even when the spiral unit 60 cannot be rotated due to a fault, etc., the insertion section 12 can easily be drawn out of the duct.

The distal end of the distal-side taper portion 156 is formed to have a small stepped portion relative to the outer surface of the insertion section 12. In addition, the proximal end of the proximal-side taper portion 158 is formed to have a small stepped portion relative to the outer surface of the insertion section 12. Thus, for example, even at a part where the transverse cross-sectional area of the inner wall of the duct steeply changes from a large state to a small state, when the insertion section 12 is inserted into the duct in the distal-end direction C1, it is possible to prevent as much as possible the inner wall of the duct from being caught on a boundary between the distal end of the distal-side taper portion 156 and the outer surface of the insertion section 12. Similarly, for example, even at a part where the transverse cross-sectional area of the duct steeply changes from a large state to a small state, when the insertion section 12 is drawn out from the duct in the proximal-end direction C2, it is possible to prevent as much as possible the inner wall of the duct from being caught on a boundary between the proximal end of the proximal-side taper portion 158 and the outer surface of the insertion section 12. Thus, in the state in which the spiral unit 60 according to this embodiment is properly mounted on the insertion section 12, the end portion of the spiral unit 60 can be prevented from being caught on the inner peripheral surface of the duct.

The spiral fin 154, in particular, the insertion assisting fin portion 188 of the distal-side taper portion 156, has a spiral radius which gradually increases from the distal end toward the proximal end side. Thus, the distal end of the spiral unit 60 is easily inserted in the inner wall of the duct. When the distal end of the spiral unit 60 is inserted, the spiral radius of the spiral unit 60, which is in contact with the inner wall of the duct, becomes larger by the rotation of the spiral unit 60 about the longitudinal axis C. Thus, the pushing force on the inner wall of the duct can gradually be increased. Therefore, the loss of driving force of the motor 132 can be minimized at a time of insertion into the duct by the rotation of the spiral unit 60. In addition, the insertion assisting fin portion 188 can assist in inserting the distal end of the spiral unit 60 into the depth side of the duct.

Besides, the spiral fin 154, in particular, the insertion assisting fin portion 188 of the proximal-side taper portion 158, has a spiral radius which gradually increases from the proximal end toward the distal end side. Thus, the spiral unit 60 is easily drawn out of the inner wall of the duct. When the spiral unit 60 is drawn out, the spiral radius of the spiral unit 60, which is in contact with the inner wall of the duct, becomes larger by the rotation of the spiral unit 60 about the longitudinal axis C. Thus, the pushing force on the inner wall of the duct can gradually be increased. Therefore, the loss of driving force of the motor 132 can be minimized at a time of draw-out from the duct by the rotation of the spiral unit 60. In addition, the insertion assisting fin portion 188 can assist in drawing out the proximal end of the spiral unit 60 to the near side of the duct. In short, the insertion assisting fin portion 188 of the proximal-side taper portion 158 functions as a draw-out assisting fin portion.

In this manner, when the insertion assisting fin portion 188 is provided on the distal-side taper portion 156, the insertion assisting fin portion 188 can assist in inserting the distal end of the spiral unit 60 into the depth side of the duct. When the insertion assisting fin portion 188 is provided on the proximal-side taper portion 158, the insertion assisting fin portion 188 can assist in drawing out the proximal end of the spiral unit 60 to the near side of the duct. Therefore, depending on the position where the fin portion 188 is mounted on the tube body 152, the fin portion 188 functions as an insertion/draw-out assisting fin portion which assists in insertion and draw-out in/from the duct.

In addition, it is possible to ignore a stepped part between the insertion assisting fin portion 188 of the distal-side taper portion 156 and the main fin portion 172 when the insertion section 12 is inserted into the duct. It is possible to ignore a stepped part between the insertion assisting fin portion 188 of the proximal-side taper portion 158 and the main fin portion 172 when the insertion section 12 is drawn out of the duct. Therefore, it is possible to prevent as much as possible the insertion section 12 from being caught on the inner wall of the duct.

Next, a second embodiment is described with reference to FIG. 9A to FIG. 11B. This embodiment is a modification of the first embodiment.

In this embodiment, as illustrated in FIG. 9A and 9B, the fin connector 186 is formed in an annular shape.

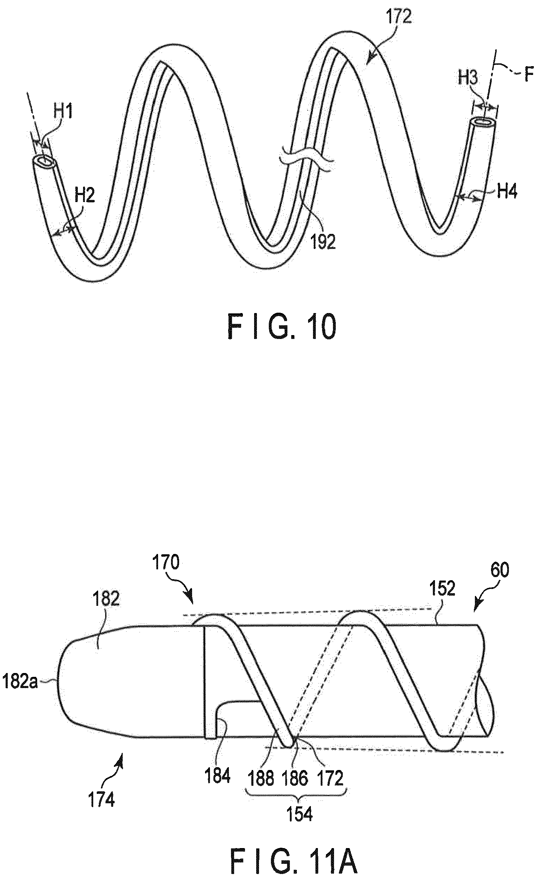

As illustrated in FIG. 10 to FIG. 11B, the main fin portion 172 is formed to have a fin height which decreases toward the distal end side thereof, relative to the outer surface of the tube body 152. Specifically, a fin height H1 of the distal end portion of the main fin portion 172 is less than a fin height H2 of the proximal end portion thereof. The insertion assisting fin portion 188 is formed to have a fin height which decreases toward the distal end side relative to the outer surface of the spiral cap 174. In other words, the insertion assisting fin portion 188 is formed such that the fin height of the insertion assisting fin portion 188 increases from the outer surface of the spiral cap 174 toward the tube body 152.

As regards the proximal end portion of the main fin portion 172, too, the main fin portion 172 is formed to have a fin height which decreases toward the proximal end side thereof, relative to the outer surface of the tube body 152. Specifically, a fin height H3 of the proximal end portion of the main fin portion 172 is less than a fin height H4 of the distal end portion thereof.

Here, the fin connector 186 of the distal-side taper portion 156 is formed to have a space which becomes narrower toward the distal end side. Thus, when the distal end portion of the main fin portion 172 is fitted into the annular fin connector 186, the distal end of the main fin portion 172 can be introduced deep into the fin connector 186 (in a direction closer to the distal end of the spiral cap 174). In the meantime, when the distal end portion of the main fin portion 172 is fitted into the annular fin connector 186, it is preferable to use an adhesive or the like.

In addition, the fin connector 186 of the proximal-side taper portion 158 is formed to have a space which becomes narrower toward the proximal end side. Thus, when the proximal end portion of the main fin portion 172 is fitted into the annular fin connector 186, the proximal end of the main fin portion 172 can be introduced deep into the fin connector 186 (in a direction closer to the proximal end of the spiral cap 174).

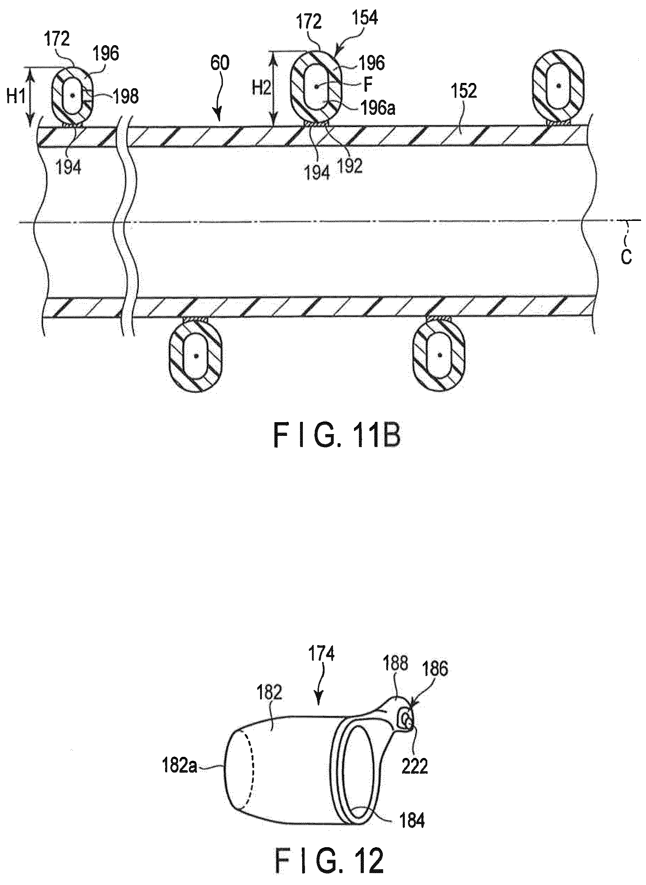

Besides, according to the spiral unit 60 of this embodiment, the same advantageous effects as described in the first embodiment can be obtained. As illustrated in FIG. 11A and 11B, the spiral fin 154 has a spiral radius which gradually increases from the distal end to the proximal end side thereof, by the insertion assistance fin portion 188 and main fin portion 172. Thus, when the insertion section 12 is inserted into the duct, the outer surface of the spiral fin 154 can easily be abutted on the inner wall of the duct from the distal end toward proximal end side thereof.

When the proximal-side taper portion 158 is formed like the distal-side taper portion 156, the spiral fin 154 has a spiral radius (a projection height (fin height) relative to the outer surface of the spiral cap 174 and tube body 152) which gradually increases from the proximal end to distal end side thereof, by the insertion assistance fin portion 188 and main fin portion 172. Thus, when the insertion section 12 is drawn out from the duct, the outer surface of the spiral fin 154 can easily be abutted on the inner wall of the duct from the proximal end toward distal end side thereof.

When the spiral unit 60 is formed in this manner, the fin height of the spiral fin 154 can be varied more gently than the fin height described in the first embodiment, between the distal end and proximal end of the spiral unit 60.

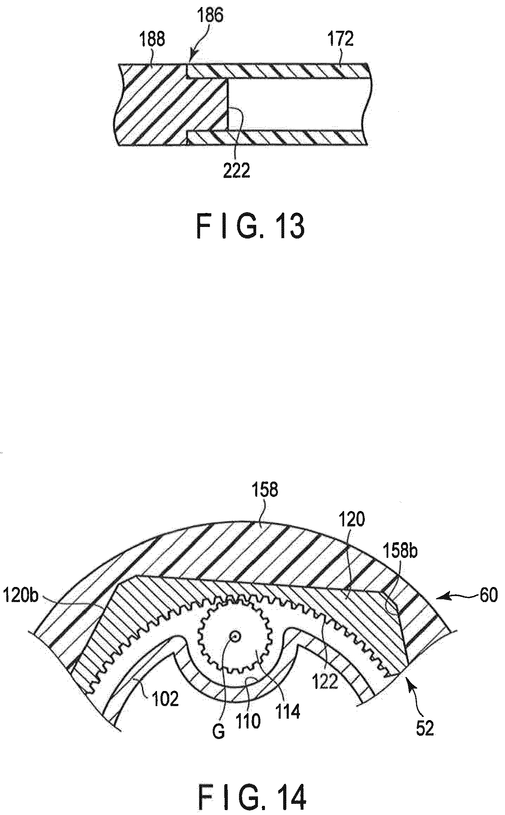

Next, a third embodiment is described with reference to FIG. 12 and FIG. 13. This embodiment is a modification of the first and second embodiments.

In this embodiment, a projection portion 222 is formed on the fin connector 186 along the fin axis F of the insertion assisting fin portion 188. An outer surface of the projection portion 222 is fitted on an inner wall of the annular wall 196 of the main fin portion 172. Incidentally, the outer surface of the projection portion 222 and the inner wall of the annular wall 196 may be adhered.

In the meantime, as illustrated in FIG. 13, it is preferable that, when the distal end of the main fin portion 172 is abutted on the proximal end of the fin connector 186, the main fin portion 172 and fin connector 186 are flush with each other at the boundary therebetween. By this formation, the number of stepped parts can be decreased over the entirety of the spiral fin 154.

Besides, according to the spiral unit 60 of this embodiment, the same advantageous effects as described in the first embodiment can be obtained. Accordingly, when the spiral unit 60 is inserted/drawn out in/from the duct, smoother movement can be realized than has been described in the first embodiment.

In the meantime, in the above-described first to third embodiments, the endoscope 10 including the observation optical system and illumination optical system was described as the introducing device for various ducts. A similar spiral unit 60 can be disposed on a catheter which includes neither an observation optical system nor illumination optical system as an introducing device for various ducts,

In the first to third embodiments, as illustrated in FIG. 2 and FIG. 3, the driving gear 114 is meshed with the relay gear 116, and the relay gear 116 is meshed with the inner peripheral gear portion 122 of the rotary cylindrical member 120, so that a driving force is transmitted from the motor 132 to the rotary cylindrical member 120. Of these parts, the relay gear 116 is not always necessary. Specifically, such a configuration is also preferable that a driving force is directly transmitted from the driving gear 114 to the inner peripheral gear portion 122 of the rotary cylindrical member 120.

Besides, in the first to third embodiments, as illustrated in FIG. 2 to FIG. 4, the description was given of the example in which one inside roller 124a is disposed between two outside rollers 162a and 162b, which are one of three pairs. In an example illustrated in FIG. 10, these rollers 124a to 124c and 162a to 162f are removed. In the example illustrated in FIG. 14, an outer peripheral surface 120b of the rotary cylindrical member 120 and an inner peripheral surface 158b of the proximal-side taper portion 158 of the spiral unit 60 are formed in mutually engageable shapes. Thus, the outer peripheral surface 120b of the rotary cylindrical member 120 is engaged with the inner peripheral surface 158b of the proximal-side taper portion 158 of the spiral unit 60, thereby transmitting the driving force of the motor 132 from the driving gear 114 to the inner peripheral gear portion 122 of the rotary cylindrical member 120. Thus, the spiral unit 60 can be rotated about the longitudinal axis C.

Although some embodiments have been concretely described with reference to the drawings, the present invention is not limited to the above-described embodiments and includes any embodiment which is implemented without departing from the spirit of the invention.

## Claims

1. A spiral cap comprising:
a cap body;
a coupling portion provided on the cap body and coupled to an end portion of a tube body which is rotatably attached to an insertion section of an introducing device;
a fin connector to which an end portion of a main fin portion is connected, the main fin portion being provided on an outer surface of the tube body in a spiral shape about a longitudinal axis of the tube body; and
an insertion assisting fin portion provided on an outer surface of the cap body and forming a fin in cooperation with the main fin portion connected to the fin connector.

2. The spiral cap according to claim 1, wherein the insertion assisting fin portion is formed such that a fin height of the insertion assisting fin portion becomes higher toward the fin connector from the outer surface of the cap body.

3. The spiral cap according to claim 1, wherein the insertion assisting fin portion is formed integral with the fin connector.

4. The spiral cap according to claim 1, wherein the insertion assisting fin portion and the cap body are formed integral.

5. The spiral cap according to claim 1, wherein:
the fin connector is provided on the insertion assisting fin portion, and
the end portion of the main fin portion is fitted and connected to the fin connector.

6. The spiral cap according to claim 1, wherein the cap body includes a through-hole for insertion of the insertion section along the longitudinal axis.

7. The spiral cap according to claim 1, wherein the outer surface of the cap body is formed in a taper shape.

8. A cap unit comprising:
the spiral cap according to claim 1; and
the main fin portion with an end portion coupled to the fin connector.

9. The cap unit according to claim 8, wherein:
the main fin portion includes an annular wall which forms a hollow section therein,
the fin connector is formed integral with the insertion assisting fin portion, and formed such that an end portion of the main fin portion is fitted to the fin connector, and
the insertion assisting fin portion includes an opening formed at a position displaced from a farthest position in a radial direction of the longitudinal axis of the tube body, such that the opening communicates with the hollow section of the main fin portion which is fitted to the fin connector.

10. The cap unit according to claim 8, wherein
the main fin portion includes an annular wall which forms a hollow section therein, and
the annular wall includes an opening formed at a position displaced from a farthest position in a radial direction of the longitudinal axis of the tube body, the opening communicating with the hollow section through the annular wall.

11. A spiral unit rotatably attached to an insertion section having a longitudinal axis, comprising:
the cap unit according to claim 8; and
the tube body to which the cap unit is attached, the tube body having an outer surface on which the main fin portion is fixed.

12. An introducing device for various ducts, comprising:
the insertion section provided to extend along a longitudinal axis, and inserted into a duct from a distal end of the insertion section; and
the spiral unit according to claim 11, which is mounted on an outer periphery of the insertion section and is rotatable about the longitudinal axis relative to the insertion section by driving of a driving member.
